(19) **Europäisches Patentamt / European Patent Office / Office européen des brevets**

(11) **EP 1 602 377 B1**

(12) **EUROPEAN PATENT SPECIFICATION**

(45) Date of publication and mention
of the grant of the patent:
**10.08.2011 Bulletin 2011/32**

(21) Application number: **04717235.8**

(22) Date of filing: **04.03.2004**

(51) Int Cl.:
*A61K 35/74* (2006.01)   *A61K 31/716* (2006.01)
*A61P 1/00* (2006.01)   *A61P 37/04* (2006.01)
*A61P 17/16* (2006.01)

(86) International application number:
**PCT/JP2004/002780**

(87) International publication number:
**WO 2004/078188 (16.09.2004 Gazette 2004/38)**

(54) **COMPOSITION CONTAINING BETA-GLUCAN AND CONSTIPATION-RELIEVING DRUG, IMMUNOPOTENTIATOR AND SKIN MOISTENING AGENT USING THE COMPOSITION**

ZUSAMMENSETZUNG MIT BETA-GLUCAN UND OBSTIPATIONSLINDERNDES MITTEL, IMMUNOPOTENTIATOR UND HAUTBEFEUCHTUNGSMITTEL UNTER VERWENDUNG DER ZUSAMMENSETZUNG

COMPOSITION CONTENANT DU BETA-GLUCANE ET MEDICAMENT DE SOULAGEMENT DE LA CONSTIPATION, IMMUNOSTIMULANT ET AGENT D'HYDRATATION DE LA PEAU UTILISANT UNE TELLE COMPOSITION

(84) Designated Contracting States:
**AT BE BG CH CY CZ DE DK EE ES FI FR GB GR HU IE IT LI LU MC NL PL PT RO SE SI SK TR**

(30) Priority: **07.03.2003 JP 2003061382**
**07.03.2003 JP 2003061379**
**05.02.2004 JP 2004028965**

(43) Date of publication of application:
**07.12.2005 Bulletin 2005/49**

(73) Proprietor: **Aureo Co., Ltd.**
**Tokyo 108-0071 (JP)**

(72) Inventors:
• **MORIYA, Naoyuki,**
c/o Aureo Co., Ltd.
**Tokyo 108-0071 (JP)**
• **MORIYA, Yukiko,**
c/o Aureo Co., Ltd.
**Tokyo 108-0071 (JP)**
• **SUZUKI, Kenji**
Itabashi-ku,
**Tokyo 173-0012 (JP)**

(74) Representative: **Beacham, Annabel Rose et al**
**Dehns**
**St Bride's House**
**10 Salisbury Square**
**London**
**EC4Y 8JD (GB)**

(56) References cited:
**WO-A1-98/26787          DE-U1- 20 202 562**
**JP-A- 1 137 990          JP-A- 10 310 515**
**JP-A- 61 146 192          JP-A- 62 205 008**
**JP-A- 2001 048 796          JP-A- 2001 231 589**
**JP-A- 2001 323 001          JP-A- 2001 354 570**
**JP-A- 2002 238 494          JP-A- 2002 531 510**
**JP-A- 2003 040 785          JP-A- 2004 121 192**

• **DATABASE WPI Week 200350 Thomson Scientific, London, GB; AN 2003-527661 XP002520808 & JP 2003 040785 A (COMBI CORP) 13 February 2003 (2003-02-13)**
• **DATABASE WPI Week 200169 Thomson Scientific, London, GB; AN 2001-605309 XP002520809 & JP 2001 190231 A (ORIENTAL YEAST CO LTD) 17 July 2001 (2001-07-17)**
• **DATABASE WPI Week 200346 Thomson Scientific, London, GB; AN 2003-485823 XP002521596 & JP 2002 335926 A (IKKO KAGAKU KK) 26 November 2002 (2002-11-26)**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

- DATABASE WPI Week 198742 Thomson Scientific, London, GB; AN 1987-294398 XP002521601 & JP 62 205008 A (BIO BY DAIMARU KK) 9 September 1987 (1987-09-09)
- DATABASE WPI Week 198633 Thomson Scientific, London, GB; AN 1986-215130 XP002521602 & JP 61 146192 A (SHINOHARA S) 3 July 1986 (1986-07-03)

- DATABASE WPI Week 200126 Thomson Scientific, London, GB; AN 2001-252979 XP002521605 & JP 2001 048796 A (ADVANCE CO LTD) 20 February 2001 (2001-02-20)

**Description**

Technical Field

The present invention relates to a composition containing β-glucan, which contains as active ingredients a culture containing β-1, 3-1, 6-glucan of a bacterium belonging to the genus *Aureobasidium sp.* and lactic acid bacterium *Enterococcus faecalis* cells that are killed by a heat treatment. The present invention further relates to a drug for use in relieving constipation, a drug for use as an immunopotentiator, and a skin moisturizer using the composition.

Background Art

It is known that β-1,3-1,6-glucan produced by a bacterium belonging to the genus *Aureobasidium sp.* (commonly known as black yeast) has various physiological activities such as an immunopotentiating action, antitumor activity, cancer cell proliferation suppressing action, antiallergic action, antiinflammatory action, hypocholesterolemic action, antithrombotic action, dietary fiber action, antihypertensive action, hypoglycemic action, and increased hepatic function. Accordingly, many attempts have been performed to use β-1,3-1,6-glucan for a functional material, pharmaceutical product, etc.

For example, JP-S57-149301-A discloses an antiflatulent and other medical drugs each containing as a principal ingredient a polysaccharide produced by a bacterium belonging to the genus *Aureobasidium sp.* of Deuteromycetes dematiaceous family (Bikoken: National Institute of Bioscience & Human-Technology, Depository No. 4257).

JP-H5-4063-B discloses a method of manufacturing a food or beverage containing as principal ingredients fructo-oligosaccharides and β-1,3-1,6-glucan. JP-H5-4063-B describes that the food or beverage can be used in a health-maintenance beverage (proliferation of Bifidobacterium in the intestine, prevention of constipation, immunopotentiation), antiflatulent, etc.

JP-2002-335926-A discloses a composition containing β-1,3-1,6-glucan and an apple extract, and describes that the composition is useful for a beverage or a skin liniment. JP-2002-335926-A also describes that the beverage can be expected to have a decreasing effect on various allergic symptoms, relieving effect on immune abnormality diseases, cancer suppressing effect, relieving effect on angiopathic diseases, relieving effect on viral diseases, relieving effect on urinary system diseases, and relieving effect on digestive system diseases such as constipation and diarrhea.

JP-H6-340701-A describes that hyperbranched β-glucan contained in the supernatant of cultured composition obtained by culturing of *Aureobasidium pullulans* IFO 4466 strain, having a number average molecular weight of 10,000 to 5,000,000, and consisting of as a main chain β-1,3 bonded glucose residues and as major side chains branched chains of β-1,6 bonded glucose residues, exhibits a high antitumor activity and immunopotentiating activity via oral administration, and is useful for a medical drug, food additive, feed additive, etc.

JP-2002-204687-A describes that a cultured composition, containing as a principal ingredient β-1,3-1,6-glucan obtained by culturing of Aureobasidium, can be applied as a pharmaceutical product for various diseases.

JP-S62-205008-A discloses an additive for a cosmetic product, etc. containing β-1,3-1,6-glucan in the bonding pattern. Further, JP-S62-205008-A describes that, in applying an aqueous solution of β-1,3-1,6-glucan without modification to the skin or hair, the pellicle formability and moisturizing ability for the skin or hair are superior to those of conventional cosmetics, styling agents, and ointment additives.

JP-10-310515-A discloses a bathwater additive that is characterized by containing at least one species of extracellular homopolysaccharide produced by a bacterium or containing the extracellular homopolysaccharide and at least one species of amino acid. Moreover, JP-10-310515-A describes that a saccharide constituting the aforementioned extracellular homopolysaccharide is β-D-glucose, and the aforementioned β-D-glucose is β-1,3-1,6-glucan produced by a bacterium belonging to the genus *Aureobasidium.*

On the other hand, as an immunopotentiator using a lactic acid bacterium, for example, JP-2001-48796-A discloses an immunoregulator containing as a principal ingredient dead bacterium cells of *Enterococcus faecalis* AD101 strain.

Moreover, JP-2003-113114-A discloses an immunopotentiating material that is characterized by containing as active ingredients bacterium cells obtained by culturing a lactic acid bacterium belonging to the genus *Enterococcus* and *Aspergillus oryzae* in a liquid medium.

Furthermore, as a composition prepared by using β-glucan and a lactic acid bacterium concomitantly, for example, JP-2003-40785-A discloses an infection-preventing composition that is characterized by containing as active ingredients a material containing β-glucan and heat-treated bacterium cells of a lactic acid producing bacterium.

Moreover, JP-2001-323001-A discloses that water-soluble β-glucan of low molecular weight is useful for prevention of various infectious diseases or tumor generation, in which the promotion of cytokine production such as TNF-production in the body and enhancement of its action lead to an enhancement of antibody-producing ability or whole-body immunity. JP-2001-323001-A also describes that the β-glucan and a lactic acid bacterium are used concomitantly.

However, since a use singly of β-1,3-1,6-glucan produced by a bacterium belonging to the genus *Aureobasidium sp.* (commonly known as black yeast) is not sufficient for various physiologically active effects above, a material having higher efficiency has been required.

Disclosure of the Invention

An object of the present invention is to provide a composition containing β-glucan in which the physiologically active effects of β-1,3-1,6-glucan contained in the cultured composition obtained by culturing of a bacterium belonging to the genus *Aureobasidium sp.*; and lactic acid bacterium *Enterococcus faecalis* cells that are killed by a heat treatment, are further enhanced, and to provide a drug for use in relieving constipation, a drug for use as an immunopotentiator, and a skin moisturizer using the composition.

In order to attain the above-mentioned object, according to one aspect of the present invention, there is provided a composition containing β-glucan, which is characterized by comprising as active ingredients: a cultured composition containing β-1,3-1,6-glucan obtained by culturing a bacterium belonging to a genus *Aureobasidium* sp.; and lactic acid bacterium *Enterococcus faecalis* cells that are killed by a heat treatment cells.

The composition containing β-glucan of the present invention contains the cultured composition containing as an active ingredient β-1,3-1,6-glucan obtained by culturing the bacterium belonging to the genus *Aureobasidium sp.*, so that not only β-1,3-1,6-glucan but also various useful ingredients contained in the cultured composition can be utilized without loss. Moreover, the lactic acid bacterium cells are contained as active ingredients, so that various physiologically active effects can be expected due to synergistic effects of the aforementioned useful ingredients contained in the cultured composition and lactic acid bacterium cells. Furthermore, since each of the above-mentioned ingredients is derived from natural product used in foods, beverages, and the like, those ingredients are highly safe.

In the above-mentioned present invention, *Aureobasidium pullulans* M-1 (FERM BP-08615) is preferable as the aforementioned bacterium belonging to the genus *Aureobasidium sp.*. According to this embodiment, β-1,3-1,6-glucan having a higher physiological activity can easily be prepared.

Moreover, the content of the aforementioned cultured composition in terms of β-1,3-1,6-glucan in solid matters of the composition preferably ranges from 1 to 80%by mass, while the content of the aforementioned lactic acid bacterium cells in solid matters of the composition preferably ranges from 4 to 95% by mass.

According to those embodiments, synergistic effects on physiological activities due to β-1,3-1,6-glucan and lactic acid bacterium cells can further be expected.

The lactic acid bacterium according to the present invention can be added to various manufactured goods that need heat treatment. Moreover, because of high preservation stability, it has extremely high safety when used as a material of foods, beverages, pharmaceutical products, etc.

According to another aspect of the present invention, there is provided a drug for use in relieving constipation containing the aforementioned composition containing β-glucan as an active ingredient.

The drug for use in relieving constipation of the present invention contains the above-mentioned composition containing β-glucan as an active ingredient, so that excellent constipation-relieving effects can be expected due to synergistic effects between lactic acid bacterium *Enterococcus faecalis* cells that are killed by a heat treatment and a variety of useful ingredients contained in the cultured composition containing β-1,3-1,6-glucan obtained by culturing a bacterium belonging to the genus *Aureobasidium sp.*.

According to another aspect of the present invention, there is provided a drug for use as an immunopotentiator containing the aforementioned composition containing β-glucan as an active ingredient.

The drug for use as an immunopotentiator of the present invention contains the above-mentioned composition containing β-glucan as an active ingredient, so that excellent immunopotentiating effects can be expected due to synergistic effects between lactic acid bacterium *Enterococcus faecalis* cells that are killed by a heat treatment and a variety of useful ingredients contained in the cultured composition containing β-1,3-1,6-glucan obtained by culturing a bacterium belonging to the genus *Aureobasidium sp.*.

According to another aspect of the present invention, there is provided a composition for use as a skin moisturizer containing the aforementioned composition containing β-glucan as an active ingredient.

The composition for use as a skin moisturizer of the present invention contains the above-mentioned composition containing β-glucan as an active ingredient, so that a skin moisturizer having an excellent long-lasting moisturizing effect and sense of use can be provided due to synergistic effects between lactic acid bacterium *Enterococcus faecalis* cells that are killed by a heat treatment and a variety of useful ingredients contained in the cultured composition containing β-1,3-1,6-glucan obtained by culturing a bacterium belonging to the genus *Aureobasidium sp.*.

Brief Description of the Drawings

Fig. 1 is a graph showing a relationship between a survival rate in days after inoculation of *Listeria monocytogenes* and a test substance.

Fig. 2 is a graph showing an effect of each test substance on the survival rate after inoculation of *Listeria monocytogenes.*

Fig. 3 is a graph showing a relationship between an average duration of survival after inoculation of *Listeria monocytogenes* and a test substance.

Fig. 4 is a graph showing an effect of each test substance on the bacterial count in the spleen in days after inoculation of *Listeria monocytogenes.*

Fig. 5 shows graphs (photographs) showing results obtained by analysis using a flow cytometer of molecules on the cell surface after inoculation of *Listeria monocytogenes.*

Fig. 6 is a graph showing results obtained by a measurement of change with time in water content in corneal layer of epidermis (skin surface conductance μS) of Subject 1 (twenties female).

Fig. 7 is a graph showing results obtained by a measurement of change with time in water content in corneal layer of epidermis (skin surface conductance μS) of Subject 2 (twenties female).

Fig. 8 is a graph showing results obtained by a measurement of change with time in water content in corneal layer of epidermis (skin surface conductance μS) of Subject 3 (thirties female).

Fig. 9 is a graph showing results obtained by a measurement of change with time in water content in corneal layer of epidermis (skin surface conductance μS) of Subject 4 (forties female).

Fig. 10 is a graph showing results obtained by a measurement of change with time in water content in corneal layer of epidermis (skin surface conductance uS) of Subject 5 (fifties female).

Best Mode for carrying out the Invention

In the present invention, as a cultured composition obtained by culturing a bacterium belonging to the genus *Aureobasidium sp.* (hereinafter, simply referred to as a cultured composition), there may be used a cultured composition itself obtained by culturing of a bacterium that belongs to the genus *Aureobasidium sp.* and has a β-1,3-1,6-glucan producing ability, a concentrated solution of the cultured composition, a solid matter obtained by removing water from the cultured composition, or the like. Preferably, the cultured composition itself or the concentrated solution of the cultured composition is used. In that case, the concentration of the solid matter of the composition is preferably 0.5 to 5% by mass, more preferably 1 to 3% by mass.

As the above-mentioned bacterium belonging to the genus *Aureobasidium sp.,* there may be used the strains described in, for example, JP-S57-149301-A, JP-H5-4063-B, or JP-2002-335926-A. In the present invention, *Aureobasidium pullulans* M-1 (Independent Administrative Institution, National Institute of Advanced Industrial Science and Technology, International Patent Organism Depositary Accession No. FERM BP-08615) is preferably used. Note that, in the present invention, β-1,3-1,6-glucan means one having a structure in which glucose is branched with β-1,6 bonds from a main chain formed by glucose with β-1,3 bonds.

The culture of the above-mentioned bacterium belonging to the genus *Aureobasidium sp.* can be performed in accordance with a known method (see JP-S57-149301-A, etc.). Specifically, the bacterium is inoculated in a medium (pH 5.2 to 6.0) containing 0.5 to 5.0% by mass of carbon source (sucrose), 0.1% by mass of N source and other trace substances (for example, vitamins or minerals). The culture is performed at a temperature of 20 to 30°C for 2 to 7 days with aeration, preferably with aeration and stirring. Theviscosity of the cultured composition becomes higher as β-1,3-1,6-glucan is produced, resulting in a gel-like composition having an increased viscosity. The cultured composition thus obtained generally contains 0.6 to 1.8% by mass of a solid matter, and the solid matter contains 5 to 80% by mass of β-1,3-1, 6-glucan. Moreover, the solid matter contains not only β-1,3-1,6-glucan but also other useful ingredients such as phosphorous, potassium, magnesium, and vitamin C, that are ingredients useful in absorption of the glucan, so that the physiologically active effects of β-1,3-1,6-glucan can be exerted efficiently.

In the present invention, a cultured composition to be used contains in the solid matter 1% by mass or more, preferably 5% by mass or more, more preferably 10% by mass or more, particularly preferably 20% by mass or more of β-1,3-1,6-glucan. If the concentration of β-1,3-1,6-glucan in the cultured composition is too low, the physiologically active effects of the glucan cannot be fully expected.

Note that β-1,3-1,6-glucan can be quantified in accordance with, for example, the following method. Specifically, a cultured composition is subjected to enzyme treatment with amylase, amyloglucosidase, protease, etc., and proteins and α-glucan such as pullulans are removed, followed by ethanol precipitation. Furthermore, filtration is performed using a glass filter, to thereby yield a resultant of substances with high-molecular weight. In such step, the resultant of substances with high-molecular weight is sufficiently washed with 80% ethanol in order to remove substances with low-molecular weight including monosaccharides. The washed high-molecular sample is further washed with acetone, and sulfuric acid is added thereto for hydrolysis. After hydrolysis, neutralization is performed, and the filtered solution is collected. Quantification of glucose is performed by the glucose oxidase method, and the value calculated based on the following mathematical expression 1 is defined as the glucan amount.

$$\text{Mathematical Expression 1: } \beta\text{-glucan (g/100 g)} = \text{glucose (g/100 g)} \times 0.9$$

Moreover, β-1,3-1,6-glucan can be quantified in accordance with the method described in JP-H3-48201-B. Specifically, after completion of culture, the cultured composition is sterilized, and bacterium cells are removed by centrifugation. A mixture of chloroform/butanol (10% (v/v)) is added to the resultant solution, and the mixture is shaken (Sevage method), followed by centrifugation for removing chloroform and insoluble matters. The procedure is repeated twice, and the precipitates are collected by ethanol precipitation. The precipitates are dissolved in distilled water, and pullulans are degradated by enzyme treatment. Subsequently, dialysis is performed in distilled water, and the dialyzed solution is subjected to ethanol precipitation, to thereby collect a precipitate (β-1,3-1,6-glucan), followed by calculation of the yield.

In the present invention, the cultured composition thus obtained may be used after sterilization by heating without pressure, or after sterilization by heating under pressure. Alternatively, the cultured composition may be sterilized after the separation and removal of bacterium cells by centrifugation, etc. Moreover, the cultured composition may be used after concentration or after drying, if necessary. Note that the cultured composition of a bacterium belonging to the genus *Aureobasidium sp.* is used as a food additive such as a thickener or a stabilizer, so it has high safety.

On the other hand, the lactic acid bacteria used in the present invention are *Enterococcus faecalis.*

*Enterococcus faecalis* lactic acid bacteria are easily available for a person skilled in the art.

In the present invention, examples of *Enterococcus faecalis* are ATCC 19433, ATCC 14508, ATCC 123655, IFO 16803, etc. When the above-mentioned cultured composition and *Enterococcus faecalis* are used concomitantly, synergistic effect due to those ingredients on the physiological activities (for example, constipation-relieving effect, immunopotentiating effect, and moisturizing effect) can be further expected.

In the present invention, the above-mentioned *Enterococcus faecalis* are sterilized by a heat treatment. This makes it possible to add to various products requiring heat treatment. Moreover, *Enterococcus faecalis* has high preservation stability and has extremely high safety when used as materials of foods, beverages, pharmaceutical products, etc. In particular, it is preferable when used in a skin moisturizer.

*Enterococcus faecalis* may be cultured in accordance with a conventional method. For example, from a cultured composition obtained by culturing *Enterococcus faecalis* in accordance with a conventional method, bacterium *Enterococcus faecalis* cells are collected by means of a method such as filtration or centrifugation. After washing with water, the cells are suspended in water, etc., followed by a heat treatment at 80 to 115°C for 30 minutes to 3 seconds. The heat-sterilized *Enterococcus faecalis* may be used after concentration or drying, if necessary.

The composition containing β-glucan of the present invention can be obtained by mixing and dispersing the above-mentioned heat-sterilized lactic acid bacterium *Enterococcus faecalis* cells in a sterilized cultured composition of the above-mentioned bacterium belonging to the genus *Aureobasidium sp.*. Moreover, if necessary, the composition may be formed into any one of various forms such as a tablet, capsule, powder, granule, liquid, paste, and jelly.

In the composition containing β-glucan of the present invention, the content of the above-mentioned cultured composition in terms of β-1,3-1,6-glucan preferably ranges from 1 to 80% by mass in solid matters, while the content of the above-mentioned lactic acid bacterium cells preferably ranges from 4 to 95% by mass in solid matters. Moreover, except the above-mentioned basic ingredients, the composition may arbitrarily contain fragrances, sweetenings, vitamins, minerals, oligosaccharides, polysaccharide thickeners, dextrins, plant extracts, other plant ingredients, etc.

The composition containing β-glucan of the present invention may be used without modification as a drug for use in relieving constipation, an immunopotentiator, a skin moisturizer, etc.

For example, when the composition containing β-glucan of the present invention is used as a drug for use in relieving constipation, the content of the above-mentioned cultured composition in terms of β-1,3-1,6-glucan preferably ranges from 1 to 40% by mass in solid matters, while the content of the above-mentioned lactic acid bacterium *Enterococcus faecalis* cells preferably ranges from 4 to 95% by mass in solid matters. The content of the above-mentioned cultured composition in terms of β-1,3-1,6-glucan more preferably ranges from 2 to 40% by mass, while the content of the above-mentioned lactic acid bacterium cells more preferably ranges from 10 to 95% by mass. The content of the above-mentioned cultured composition in terms of β-1,3-1,6-glucan particularly preferably ranges from 3 to 40% by mass, while the content of the above-mentioned lactic acid bacterium cells particularly preferably ranges from 30 to 95% by mass.

With respect to an effective uptake of the drug for use in relieving constipation of the present invention per day for an adult, the amount of the above-mentioned cultured composition in terms of β-1,3-1,6-glucan ranges from 0.01 to 10 g, while the amount of the above-mentioned lactic acid bacterium cells ranges from 0.01 to 10 g. Preferably, the amount of the above-mentioned cultured composition in terms of β-1,3-1,6-glucan ranges from 0.5 to 5 g, while the amount of the above-mentioned lactic acid bacterium cells ranges from 0.05 to 1 g.

The drug for use in relieving constipation of the present invention may be incorporated in various foods and beverages such as cold beverages, jelly beverages, fruit juice beverages, vegetable juices, soups, miso soups, frozen foods, and other processed foods. The amount of the above-mentioned drug of the present invention to be added in each of the above-mentioned foods and beverages may be defined based on the above-mentioned effective uptake per day for an adult. Generally, the amount is preferably 1 to 50% by mass, more preferably 10 to 20% by mass. Note that the addition method is not particularly limited, and the drug may be added from the beginning together with other materials to be used in various foods and beverages.

Moreover, when the composition containing β-glucan of the present invention is a drug for use as an immunopotentiator, the content of the above-mentioned cultured composition in terms of β-1,3-1,6-glucan preferably ranges from 5 to 80% by mass in solid matters, while the content of the above-mentioned lactic acid *Enterococcus faecalis* bacterium cells preferably ranges from 10 to 80% by mass in solid matters. The content of the above-mentioned cultured composition in terms of β-1,3-1,6-glucan more preferably ranges from 25 to 70% by mass, while the content of the above-mentioned lactic acid bacterium cells more preferably ranges from 20 to 70% by mass. The content of the above-mentioned cultured composition in terms of β-1,3-1,6-glucan particularly preferably ranges from 30 to 60% by mass, while the content of the above-mentioned lactic acid bacterium cells particularly preferably ranges from 30 to 60% by mass.

With respect to the effective uptake of the drug for use as an immunopotentiator of the present invention per day for an adult, the amount of the above-mentioned cultured composition in terms of β-1,3-1,6-glucan ranges from 0.02 to 0.50 g, while the amount of the above-mentioned lactic acid bacterium cells ranges from 0.10 to 0.90 g. Preferably, the amount of the above-mentioned cultured composition in terms of β-1,3-1,6-glucan ranges from 0.06 to 0.40 g, while the amount of the above-mentioned lactic acid bacterium cells ranges from 0.15 to 0.45 g.

The drug for use as an immunopotentiator of the present invention maybe incorporated in various foods and beverages such as cold beverages, jelly beverages, fruit juice beverages, vegetable juices, soups, miso soups, frozen foods, and other processed foods. The amount of the above-mentioned drug of the present invention to be added in each of the above-mentioned foods and beverages may be defined based on the above-mentioned effective uptake per day for an adult. Generally, the amount is preferably 0.02 to 0.50% by mass, more preferably 0.06 to 0.40% by mass in terms of β-1,3-1,6-glucan. Moreover, the amount of the lactic acid bacterium cells is preferably 0.10 to 0.90% by mass, more preferably 0.15 to 0.45% by mass. Note that the addition method is not particularly limited, and the immunopotentiator may be added from the beginning together with other materials to be used in various foods and beverages.

Moreover, when the composition containing β-glucan of the present invention is used as a skin moisturizer, the content of the above-mentioned cultured composition in terms of β-1,3-1,6-glucan preferably ranges from 1 to 40% by mass in solid matters, while the content of the heat-sterilized cells of the above-mentioned lactic acid bacterium *Enterococcus faecalis* cells preferably ranges from 4 to 95% by mass in solid matters. The content of the above-mentioned cultured composition in terms of β-1,3-1,6-glucan more preferably ranges from 2 to 40% by mass, while the content of the heat-sterilized cells of the above-mentioned lactic acid bacterium cells more preferably ranges from 10 to 95% by mass. The content of the above-mentioned cultured composition in terms of β-1,3-1,6-glucan particularly preferably ranges from 3 to 40% by mass, while the content of the heat-sterilized cells of the above-mentioned lactic acid bacterium cells particularly preferably ranges from 30 to 95% by mass. If the incorporated amount of a material containing β-glucan is too low, the moisturizing effect is not obtained sufficiently, while if the amount is too much, the fluidity (spreadability) of the product becomes lower, resulting in deterioration of the sense of use. In addition, if the incorporated amount of heat-sterilized lactic acid bacterium cells is too low, the sustainability of the moisturizing effect cannot be obtained sufficiently, while if the amount is too much, the properties of dispersion in the product becomes worse and uniform products cannot be obtained.

Although it is not clearly understood why the sustainability of the moisturizing effect is improved by a combinational use of the above-mentioned cultured composition and the above-mentioned heat-sterilized lactic acid bacterium cells, for example, heat-sterilized lactic acid bacterium cells serving as carriers probably maintain β-glucan or water. *Enterococcus faecalis* of the present invention is smaller than those of other lactic acidbacteria such as *Lactobacillus* and *Bifidobacteria,* so the number of the bacterium cells per mass of the added cells is larger, resulting in enhancement of the moisturizing effect.

The composition for use as a skin moisturizer of the present invention may be used without modification as a lotion or cosmetic liquid, or may be incorporated in various skin cosmetics (for example, milky lotion, cream, pack, etc.). The incorporated amount of the skin moisturizer in a skin cosmetic is preferably 0.5 to 50% by mass, more preferably 5 to 20% by mass.

Examples

Hereinafter, the present invention will be described specifically by way of examples. Note that, in the following descriptions, the term "%" represents "% by mass" unless otherwise specified.

Example 1

(1) Culture of *Aureobasidium*

An appropriate amount of pre-culture of *Aureobasidium pullulans* M-1 (FERM BP-08615) was inoculated in a liquid medium (pH 5.3) containing 1% of sucrose, 0.2% of ascorbic acid, and 0.2% of rice polishings, and the culture was performed with aeration and stirring at 25°C for 2 days. After completion of the culture, the cultured composition was sterilized at 121°C for 15 minutes. The solid matter content in the cultured composition was 1%, and the solid matters contained 35% of β-1,3-1,6-glucan.

(2) Culture of *Enterococcus faecalis*

An appropriate amount of pre-culture obtained by culturing *Enterococcus faecalis* (IFO 16803) in Rogosa medium at 37°C for 24 hours was inoculated in a liquidmediumcontaining 4% of yeast extracts, 3% of polypeptone, and 10% of lactose, and neutralization culture was performed at 37°C for 22 to 24 hours while the pH value of the medium was controlled to pH 6.8 to 7.0 with an aqueous solution of sodium hydroxide using a pH stat.
After completion of the culture, the bacterium cells were separated and collected using a continuous centrifuge. Thereafter, water was added thereto for diluting to the former liquid amount, and the bacterium cells were separated and collected again using the continuous centrifuge. The operations were repeated four times in all for washing the bacterium cells. Subsequently, the washed bacterium cells were suspended in an appropriate amount of water, and the mixture was sterilized at 100°C for 30 minutes. Then, the bacterium cells were dried using a spray drier, to thereby prepare powder of heat-sterilized bacterium cells ($5 \times 10^{12}$ cfu/g).

(3) Test of constipation-relieving effect

The above-mentioned Aureobasidium cultured composition obtained in the section (1) and the above-mentioned heat-sterilized bacterium cells of lactic acid bacterium obtained in the section (2) were used for a test for confirming a constipation-relieving effect in accordance with the following method.
The test period was defined as 4 weeks. To 10 volunteers suffering from constipation, no administration was performed over the first one week (the first week), lactic acid bacterium cells (200 mg/day) were administered over the next one week (the second week), the Aureobasidium cultured composition (15 ml/day) was administered over the next one week (the third week), and the Aureobasidium cultured composition (15 ml/day) and the lactic acid bacterium cells (200 mg/day) were administered over the last one week (the fourth week). Thereafter, the frequency of defecation in the administration period of each test sample (a week) was checked. The results are shown in Table 1.

[Table 1]

| | Frequency of defecation | | | |
|---|---|---|---|---|
| | First week | Second week | Third week | Fourth week |
| Subject | Before administration | Only the lactic acid bacterium cells | Only the Aureobasidium cultured composition | Aureobasidium cultured composition plus the lactic acid bacterium cells |
| 1 | 3 | 3 | 3 | 5 |
| 2 | 2 | 4 | 2 | 5 |
| 3 | 4 | 4 | 3 | 6 |
| 4 | 3 | 4 | 3 | 5 |
| 5 | 2 | 2 | 2 | 3 |
| 6 | 2 | 3 | 3 | 4 |

(continued)

| Subject | Before administration | Only the lactic acid bacterium cells | Only the Aureobasidium cultured composition | Aureobasidium cultured composition plus the lactic acid bacterium cells |
|---|---|---|---|---|
| 7 | 4 | 5 | 3 | 7 |
| 8 | 2 | 3 | 3 | 5 |
| 9 | 3 | 3 | 3 | 6 |
| 10 | 3 | 4 | 3 | 5 |
| Average | 2.8 | 3.5 | 2.8 | 5.1 |

Table 1 shows that, in the case of administration of only the lactic acid bacterium cells, there is a slight constipation-relieving effect, while in the case of administration of only the Aureobasidium cultured composition, there is little constipation-relieving effect. On the other hand, in the case of combinational administration of the Aureobasidium cultured composition and the lactic acid bacterium cells, there are increased frequencies of defecation, so it is confirmed that constipation is clearly alleviated.

Example 2

To 1 L of the Aureobasidium cultured composition obtained in Example 1 (1), 10 g of the heat-sterilized lactic acid bacterium cells obtained in Example 1 (2) was added, and the mixture was uniformly stirred, to thereby yield a composition containing β-glucan. In accordance with the following method, the composition containing β-glucan was tested for the protection effect against early infection.

(1) Measurement of survival rate

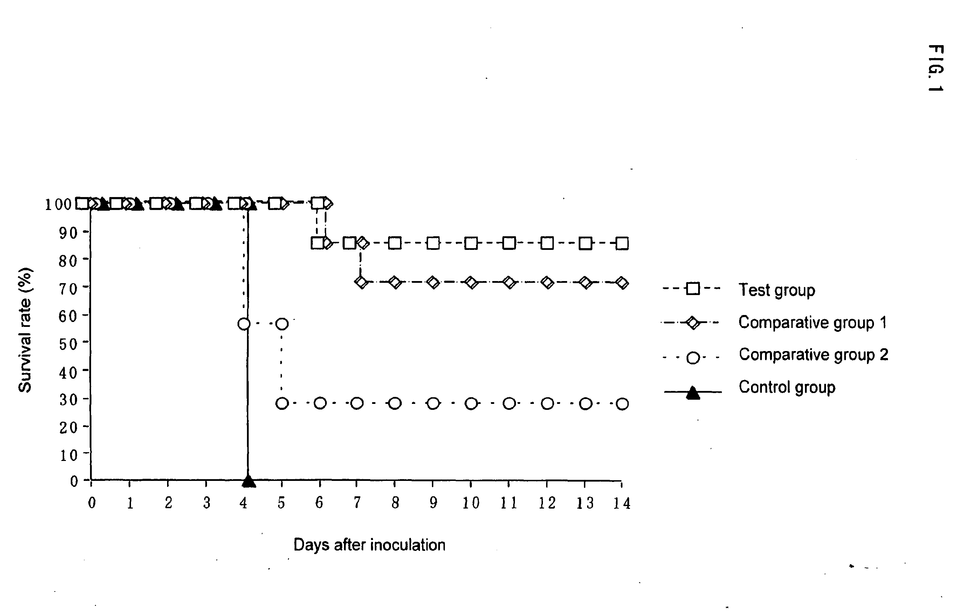

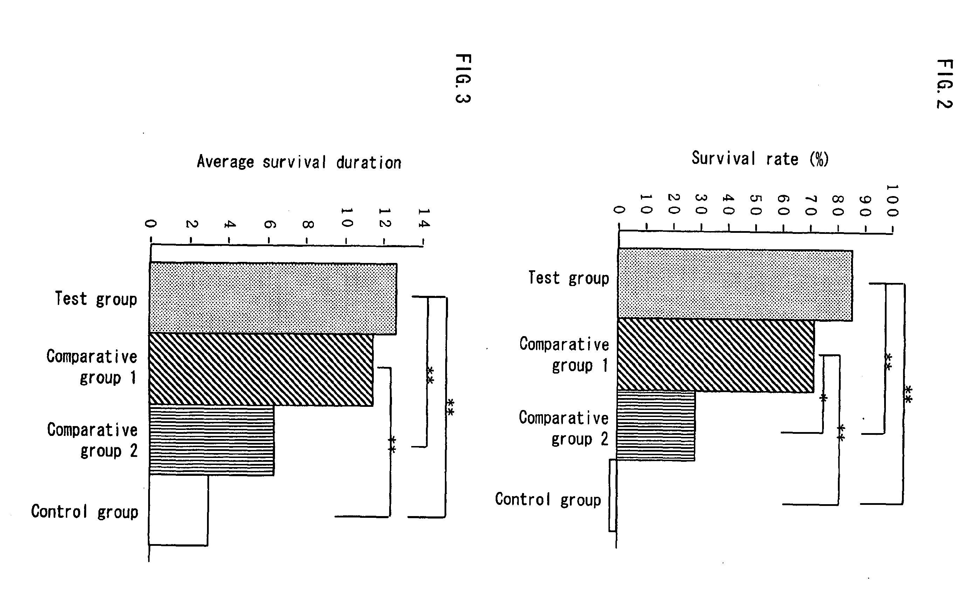

Twenty eight of BALB/c mice (7-week-old, females) (purchased from Japan SLC, Inc.), which had been subj ected to preliminary feeding for a week, were divided into 4 groups (7 mice in each group), and 200 μl of each of the following test substances was orally administered to a mouse of the respective groups once a day continuously over the test period.
Test group: the above-mentioned composition containing β-glucan
Comparative group 1: the Aureobasidium cultured composition (which is obtained in Example 1 (1))
Comparative group 2: a mixture prepared by suspending the heat-sterilized lactic acid bacterium cells (which are obtained in Example 1 (2)) in PBS (1 g bacterium cells/100 ml)
Control group: PBS
An intracellular parasitism, *Listeria monocytogenes* (EDG strain), was inoculated at a concentration of $5.4 \times 10^4$ cfu/200 μl/mouse ($2 \times LD_{50}$) via the tail vein of a mouse of each group to which each of the above-mentioned test substances had been orally administered continuously over a week. Subsequently, follow up was performed for 2 weeks, and the survival rate and average survival duration for mice of each group were calculated.
The results are shown in Table 2 and Figs. 1 to 3. Note that the mice were allowed to eat water and diet (trade name "Powder Diet CRF-1", manufactured by Oriental Yeast Co., Ltd.) freely over the test period.

[Table 2]

| Group | Survival rate | Average survival duration |
|---|---|---|
| Test group | 85.7%(6/7) | 12.7 days |
| comparative group 1 | 71.4%(5/7) | 11.5 days |
| comparative group 2 | 28.6%(2/7) | 6.4 days |
| control group | 0%(0/7) | 3.0 days |

As is clear from Table 2 and Figs. 1 to 3, in the test group, only one mouse died (on the sixth day after inoculation of bacterium), and the survival rate at the end of the test was 85.7%, while in the control group, all mice died (on the fourth day after inoculation of bacterium), and the survival rate at the end of the test was 0%. Moreover, in the comparative group 1, two of the mice died (on the sixth day (a mouse) and the seventh day (a mouse) after inoculation of bacterium), and the survival rate at the end of the test was 71.4%. Furthermore, in the comparative group 2, five of the mice died (on the fourth day (three mice) and fifth day (two mice) after inoculation of bacterium), and the survival rate at the end of the test was 28.6%. A significant difference between the test group and each of the other groups was assessed by the Mann-Whitney U test. As a result, the level of significance in the test group to one of the control group and the comparative group 2 was less than 1%.

On the other hand, the average survival duration of the test group was 12.7 days, while the average survival durations of the control group, the comparative group 1, and the comparative group 2 were 3.0 days, 11.5 days, and 6.4 days, respectively. A significant difference between the test group and each of the other groups was assessed by the Mann-Whitney U test. As a result, the level of significance in the test group to one of the control group and the comparative group 2 was less than 1%.

Those results suggested that resistance of a host against bacterium infection is enhanced by oral administration of the composition containing β-glucan of the present invention.

(2) Determination of bacterial counts in organ

The section (1) above suggested that the composition containing β-glucan of the present invention acts on the resistance against *Listeria monocytogenes* infection. Thus an analysis was performed on the change in bacterial counts in an organ with time, which is an indicator of bacterium exclusion.

Thirty of BALB/c mice (7-week-old, females), which had been subjected to preliminary feeding for a week, were used per group, and each test substance was administered in a manner similar to that in the section above. Then, a intracellular parasitism, *Listeria monocytogenes* (EDG strain), was inoculated at a concentration of $2.7 \times 10^3$ cfu/200 $\mu$l/mouse ($1/10 \times LD_{50}$) via the tail vein of a mouse of each group to which each of the above-mentioned test substances had been orally administered continuously over a week. After inoculation of bacterium, five mice of each group were sacrificed in order at a time on the first, third, fifth, seventh, and tenth day, and the spleens were collected.

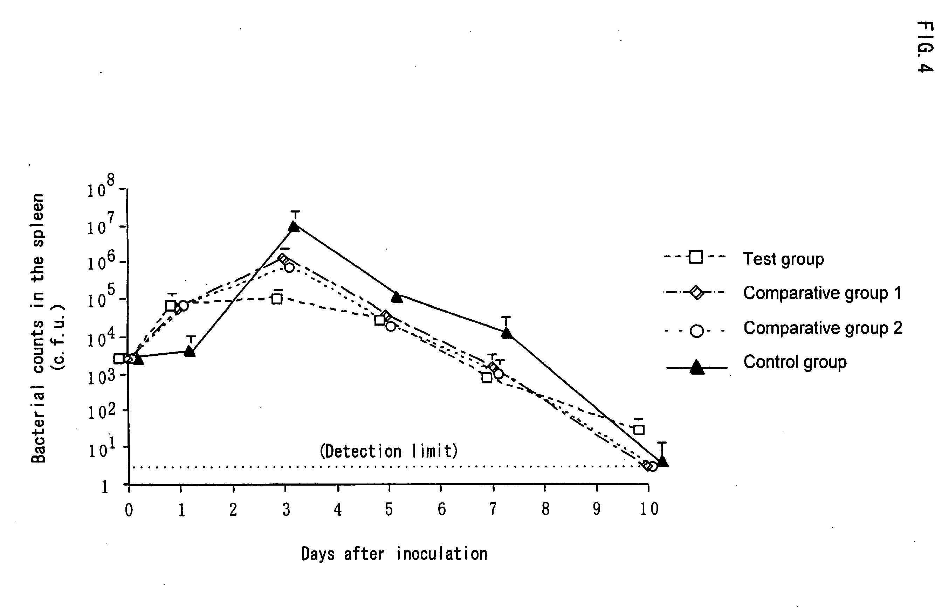

The collected spleens were homogenized with a blender, and the homogenized product was resuspended in 5 ml of PBS to prepare a concentrate mixture. The concentrate mixture was diluted by 10-fold serial dilution, and 100 $\mu$l of the concentrate mixture or each serial diluted mixture was smeared on TSA medium, followed by culturing in a 37 °C incubator for 16 hours. The number of cell colonies grown on TSA medium was determined, and bacterial counts in an organ were estimated. For the bacterial counts in an organ of each group, an average value and a standard error were calculated. The results are shown in Table 3 and Fig. 4.

[Table 3]

| Group | Days after *Listeria monocytogenes* inoculation | | | | |
|---|---|---|---|---|---|
| | 1 day | 3 days | 5 days | 7 days | 10 days |
| Test group | $6.88 \pm 8.18 \times 10^4$ | $9.79 \pm 7.76 \times 10^4$ | $2.86 \pm 1.45 \times 10^4$ | $7.24 \pm 8.78 \times 10^2$ | $2.76 \pm 2.54 \times 10^1$ |
| Comparative group 1 | $5.66 \pm 1.77 \times 10^4$ | $1.20 \pm 1.13 \times 10^6$ | $3.83 \pm 2.76 \times 10^4$ | $1.36 \pm 1.87 \times 10^3$ | Less than detection limit |
| Comparative group 2 | $7.06 \pm 0.30 \times 10^4$ | $7.12 \pm 4.06 \times 10^5$ | $2.00 \pm 1.56 \times 10^4$ | $9.75 \pm 14.9 \times 10^2$ | Less than detection limit |
| Control group | $4.37 \pm 7.47 \times 10^3$ | $1.00 \pm 1.62 \times 10^7$ | $1.14 \pm 0.85 \times 10^5$ | $1.17 \pm 2.36 \times 10^4$ | $3.75 \pm 7.50$ |

As is clear from Table 3, in the control group, the bacterial counts in the spleen reached a peak on the third day after inoculation of bacterium. After that, the bacterial counts gradually decreased, and the counts reached near the detection limit ($3.75 \pm 7.5$ CFU/spleen) on the tenth day after inoculation of bacterium. Meanwhile, in the test group, the bacterial counts increased on the first day after inoculation of bacterium, but the bacterial counts on the third day after inoculation of bacterium were about the same as those on the first day. After that, the bacterial counts decreased, and the counts reached $27 \pm 25$ CFU/spleen on the tenth day after inoculation of bacterium. In the comparative group 1 and the comparative group 2, the bacterial counts increased on the first day after inoculation of bacterium, and the bacterial counts in the spleen reached a peak on the third day after inoculation of bacterium. After that, the bacterial counts gradually decreased, and the counts were less than the detection limit (< 3. 33 CFU/spleen) on the tenth day after inoculation of bacterium.

Those results presume that the composition containing β-glucan of the present invention acts on the immune system in early infection, and the resistance against infection is possibly enhanced not by an immune system of a mature T-cell having strong aggressiveness, but by a non-specific immune system of an immature T-cell or macrophage.

(3) Analysis of cell surface molecule

In the sections (1) and (2) above, there were differences in the survival rate, survival duration, and bacterial counts in an organ, so an analysis was performed on host cells involved in infectious resistance against *Listeria monocytogenes* using a flow cytometer.

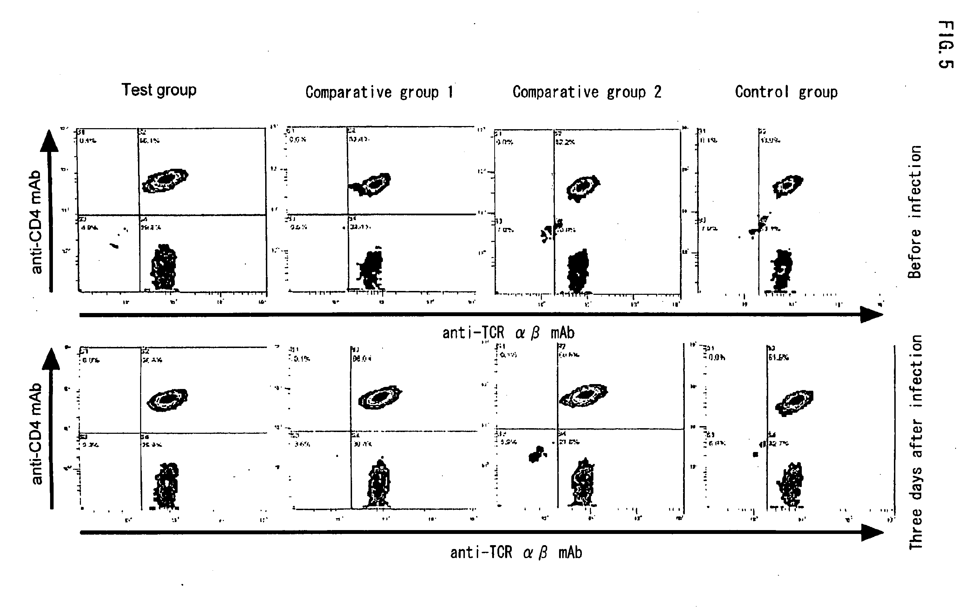

The mesenteric lymph nodes (MLN) were collected at the same time as the determination of bacterial counts in an organ described in the section (2) above. The collected MLNs were homogenized with a glass slide, and excess tissue fragments were removed with a stainless mesh. Subsequently, the lymphocytes were resuspended in the Hank's solution for FACS so as to be at a concentration of $1 \times 10^6$/ml. The lymphocytes were stained with four staining sets shown in the following (a) to (d), and analysis of cell (mainly T-cell) surface molecules was performed using a flow cytometer (Epics-XL; Beckman Coulter). The results are shown in Table 4 and Fig. 5.

(a) Cy-chrome (Cy)-labeled anti-CD3 mAb (T-cell specific recognition marker) /FITC-labeled anti-TCR αβ mAb (Type recognition marker for T-cell) /PE-labeled anti-CD4 mAb/biotin-labeled anti-TCR γδ mAb (Type recognition marker for T-cell)

(b) Cy-labeled anti-CD3 mAb/FITC-labeled anti-TCR αβ mAb/PE-labeled anti-CD4 mAb/biotin-labeled anti-CD69 mAb (early activation marker)

(c) Cy-labeled anti-CD3 mAb/FITC-labeled anti-TCR αβ mAb/PE-labeled anti-CD4 mAb/biotin-labeled anti-CD25 mAb (IL-2Rα; activation marker)

(d) Cy-labeled anti-CD3 mAb/FITC-labeled anti-TCR αβ mAb/PE-labeled anti-CD122 mAb (IL-2Rβ; activation marker) /biotin-labeled anti-CD4 mAb

[Table 4]

| Group | CD25/CD122/CD69 Expression rate (%) Days after *Listeria monocytogenes* inoculation | | | | | |
|---|---|---|---|---|---|---|
| | 0 day | 1 day | 3 days | 5 days | 7 days | 10 days |
| Test group | 20/20/20 | 21/20/20 | 24/23/35 | 20/22/20 | 24/20/20 | 19/20/20 |
| Comparative group 1 | 20/20/20 | 20/22/20 | 21/22/33 | 21/20/20 | 20/22/20 | 20/20/21 |
| Comparative group 2 | 20/20/20 | 20/20/20 | 24/20/38 | 20/23/20 | 23/19/20 | 20/18/20 |
| Control group | 20/20/21 | 20/20/19 | 22/23/28 | 21/20/20 | 20/20/21 | 20/22/20 |

As shown in Table 4 and Fig. 5, in the test group and the comparative groups 1 and 2, CD4-positive αβ-T cells increased as compared to the control group. In particular, the increasing rate in the test group was larger than those in the comparative groups 1 and 2. Note that, three days after infection of bacterium, the increasing rates of all groups were almost the same. On the other hand, with respect to the activation marker in the CD4-positive cell, CD25 molecule (IL-2Rα), CD122 molecule (IL-2Rβ), or CD69 molecule (early activation marker), it was all expressed at almost same level in every group.

Those results showed that there was no considerable change in the CD4-positive αβ-T cells before and after infection in the test group. Thus, an enhancement of functions in a population of cells that is lowly differentiated is supposed to be a cause of improvement of the resistance against infection by the composition containing β-glucan of the present invention.

The cytokines in blood were measured in accordance with the method according to a commercially available IFN-γ measuring kit (manufactured by Genzyme Corporation). As a result, as shown in Table 5, the amount of IFN-γ in the test group was high level on the third day after infection. Since no change in CD4-positive αβ-T cells was observed, it was presumed that cells that produce IFN-γ were macrophages.

[Table 5]

| Group | Produced amount of IFN-γ (units/ml) Days after *Listeria monocytogenes* inoculation | | | | | |
|---|---|---|---|---|---|---|
| | 0 day | 1 day | 3 days | 5 days | 7 days | 10 days |
| Test group | 20 | 100 | 450 | 300 | 140 | 80 |
| Comparative group 1 | 20 | 120 | 240 | 300 | 260 | 100 |
| Comparative group 2 | 20 | 140 | 360 | 320 | 140 | 100 |
| Control group | 20 | 60 | 80 | 140 | 240 | 160 |

Example 3

The Aureobasidium cultured composition obtained in Example 1 (1) and the heat-sterilized bacterium cells of lactic acid bacterium obtained in Example 1 (2) were used to prepare the following test samples, and a confirmatory test for a moisturizing effect was performed for 5 female volunteers (twenties: 2 subjects; thirties, forties, and fifties: 1 subject each).

• Test samples

1: Only Aureobasidium cultured composition
2: Aqueous suspension of 1% heat-sterilized bacterium cell of lactic acid bacterium
3: Aureobasidium cultured composition containing 1% heat-sterilized bacterium cell of lactic acid bacterium

4: Water (control)

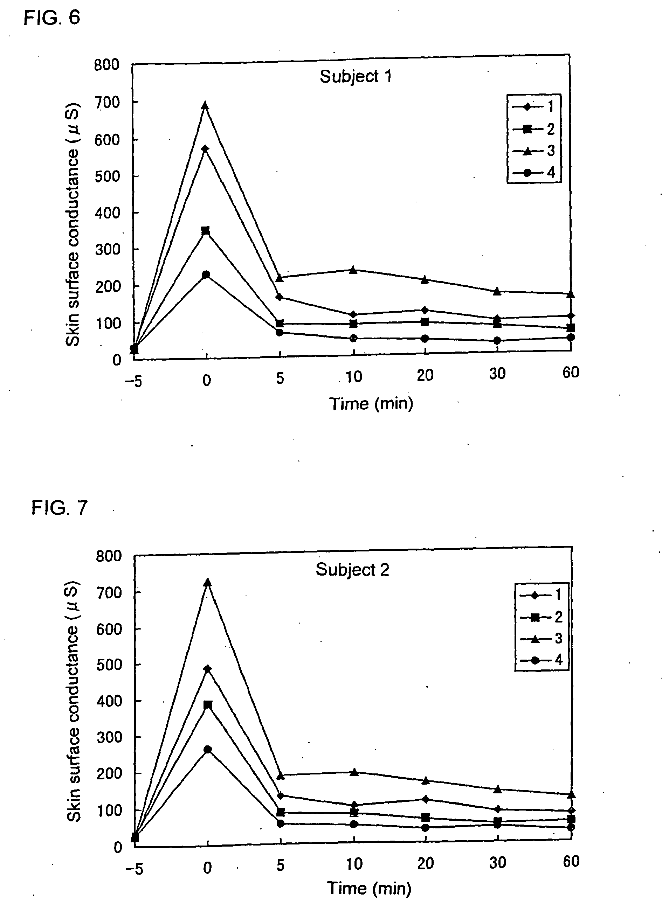

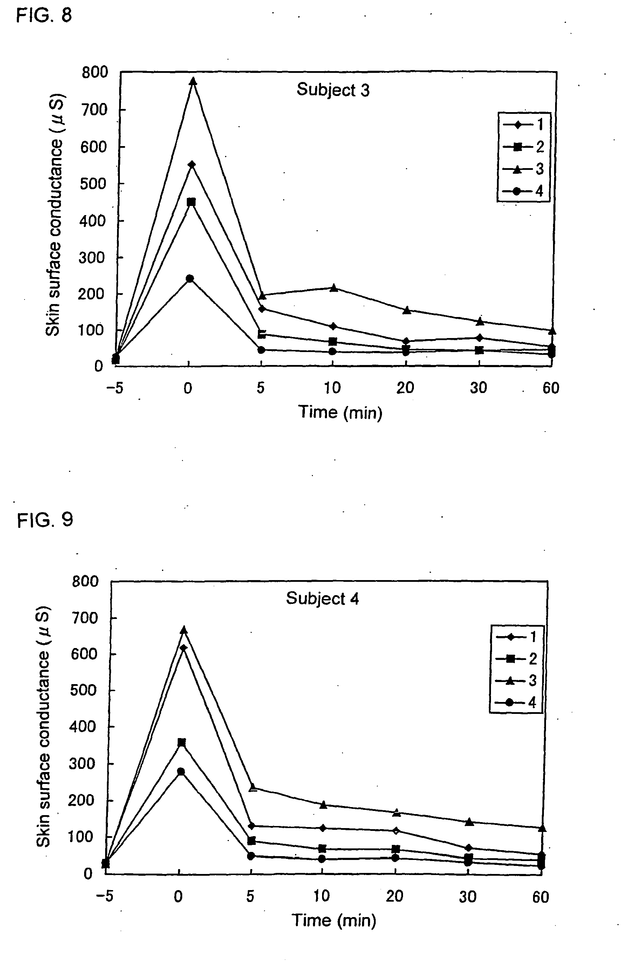

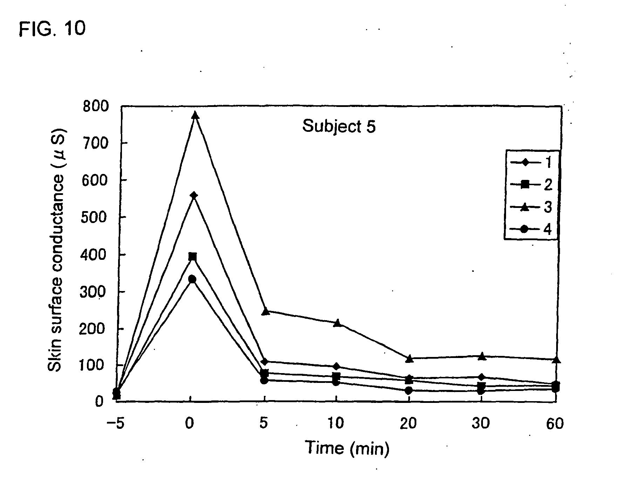

The site to be measured (area: 3 cm (width) $\times$ 10 cm (length) 5 cm below the flexion site of the medial side of the forearm) of each subject was washed with soap, and each subject kept quiet in a room with constant temperature and humidity controlled to a temperature of 18 to 20°C and a humidity of 50 to 55%. Subsequently, the above-mentioned site to be measured was divided into 4 parts, and 10 $\mu$l of each of the above-mentioned test samples 1 to 4 was applied to each part. Thereafter, the water content in corneal layer of epidermis (skin surface conductance $\mu$S) was measured with time using a measurement apparatus for the water content in corneal layer of epidermis "SKINCON-200" (trade name, manufactured by IBS Co., Ltd.) with a function according to the radio-frequency wave impedance method. Note that the water content in corneal layer of epidermis was measured 5 minutes before application of the test sample (-5 munites), immediately after application (0 minutes), and 10, 20, 30, and 60 minutes after application. The results are shown in Figs. 6 to 10. Note that, in Figs. 6 to 10, the numbers 1 to 4 correspond to the test samples 1 to 4, respectively.

Figs. 6 to 10 show that, over after time has passed from the application, in case of test sample 3, the water content in corneal layer of epidermis is maintained at a high level and the moisturizing effect is sustained, as compared to the application of test sample 1 or 2. Moreover, according to the results of the questionnaire survey for the subjects on the sense of use, they stated that the sample was nonsticky and non-oily, and they had good sense of use.

Industrial Applicability

As described above, the composition containing β-glucan of the present invention contains a cultured composition containing β-1,3-1,6-glucan obtained by culturing a bacterium belonging to the genus *Aureobasidium sp*, and lactic acid bacterium *Enterococcus faecalis* cells that are killed by a heat treatment, so that excellent physiologically active effects can be expected due to the synergistic effect of those ingredients. Accordingly, the composition can be used for a constipation-relieving drug, an immunopotentiator, a skin moisturizer, etc.

**Claims**

1.  A composition containing β-glucan, **characterized by** comprising as active ingredients: a cultured composition containing β-1,3-1,6-glucan obtained by culturing a bacterium belonging to the genus *Aureobasidium sp*.; and lactic acid bacterium *Enterococcus faecalis* cells that are killed by a heat treatment.

**2.** A composition containing β-glucan according to claim 1, wherein the bacterium belonging to the genus *Aureobasidium sp.* is *Aureobasidium pullulans* M-1 (FERM BP-08615).

**3.** A composition containing β-glucan according to claim 1 or 2, wherein a content of the cultured composition in terms of β-1,3-1,6-glucan in solid matters of the composition ranges from 1 to 80% by mass, and a content of the lactic acid bacterium cells in solid matters of the composition ranges from 4 to 95% by mass.

**4.** A drug for use in relieving constipation, comprising as an active ingredient the composition containing β-glucan according to any one of claims 1 to 3.

**5.** A drug for use as an immunopotentiator, comprising as an active ingredient the composition containing β-glucan according to any one of claims 1 to 3.

**6.** A composition for use as a skin moisturizer, comprising as an active ingredient the composition containing β-glucan according to any one of claims 1 to 3.

**7.** A composition containing β-glucan according to any one of claims 1 to 3 for use in therapy.

**8.** A composition containing β-glucan according to any one of claims 1 to 3 for use in relieving constipation.

**9.** A composition containing β-glucan according to any one of claims 1 to 3 for use as an immunopotentiator.

**10.** Use of a composition containing β-glucan according to any one of claims 1 to 3 for the manufacture of a medicament for use in the treatment of constipation or as an immunopotentiator.

**Patentansprüche**

**1.** Zusammensetzung mit β-Glucan, **dadurch gekennzeichnet, dass** sie als Wirkstoffe Folgendes umfasst: eine durch Kultivieren eines Bakteriums, das zur Gattung *Aureobasidium* sp. gehört, erhaltene Kulturzusammensetzung, die β-1,3-1,6-Glucan enthält, sowie durch Hitzebehandlung abgetötete Zellen des Milchsäurebakteriums *Enterococcus faecalis.*

**2.** Zusammensetzung mit β-Glucan nach Anspruch 1, wobei es sich bei dem zu der Gattung *Aureobasidium* sp. gehörenden Bakterium um *Aureobasidium pullulans* M-1 (FERM BP-08615) handelt.

**3.** Zusammensetzung mit β-Glucan nach Anspruch 1 oder 2, wobei ein Gehalt der Kulturzusammensetzung in Bezug auf das β-1,3-1,6-Glucan als Trockenmasse der Zusammensetzung im Bereich von 1 bis 80 Masse-% liegt und wobei ein Gehalt der Milchsäurebakteriumzellen als Trockenmasse der Zusammensetzung im Bereich von 4 bis 95 Masse-% liegt.

**4.** Arzneimittel für die Verwendung bei der Linderung von Obstipation, das als Wirkstoff die Zusammensetzung mit β-Glucan nach einem der Ansprüche 1 bis 3 enthält.

**5.** Arzneimittel zur Verwendung als Immunpotentiator, das als Wirkstoff die Zusammensetzung mit β-Glucan nach einem der Ansprüche 1 bis 3 enthält.

**6.** Zusammensetzung für die Verwendung als feuchtigkeitsspendendes Mittel für die Haut, das als Wirkstoff die Zusammensetzung mit β-Glucan nach einem der Ansprüche 1 bis 3 enthält.

**7.** Zusammensetzung mit β-Glucan nach einem der Ansprüche 1 bis 3 zur Verwendung in der Therapie.

**8.** Zusammensetzung mit β-Glucan nach einem der Ansprüche 1 bis 3 zur Verwendung bei der Linderung von Obstipation.

**9.** Zusammensetzung mit β-Glucan nach einem der Ansprüche 1 bis 3 zur Verwendung als Immunpotentiator.

**10.** Verwendung einer Zusammensetzung mit β-Glucan nach einem der Ansprüche 1 bis 3 zur Herstellung eines Arzneimittels zur Verwendung bei der Behandlung von Obstipation oder als Immunpotentiator.

**Revendications**

**1.** Composition contenant du β-glucane, **caractérisée en ce qu'**elle comprend en tant que substances actives : une composition cultivée contenant du β-1,3-1,6-glucane obtenue par culture d'une bactérie appartenant au genre *Aureobasidium sp.*; et des cellules de bactérie lactique *Enterococcus faecalis* qui son tuées par un traitement thermique.

**2.** Composition contenant du β-glucane selon la revendication 1, dans laquelle la bactérie appartenant au genre *Aureobasidium sp.* est *Aureobasidium pullulans* M-1 (FERM BP-08615).

**3.** Composition contenant du β-glucane selon la revendication 1 ou 2, dans laquelle la teneur de la composition cultivée en termes de β-1,3-1,6-glucane dans les matières solides de la composition est dans la plage de 1 à 80 % en masse, et la teneur des cellules de bactérie lactique en matières solides de la composition est dans la plage de 4 à 95 % en masse.

**4.** Médicament pour utilisation dans le traitement de la constipation, comprenant en tant que substance active la composition contenant du β-glucane selon l'une quelconque des revendications 1 à 3.

**5.** Médicament pour utilisation en tant qu'immunopotentialisateur, comprenant en tant que substance active la composition contenant du β-glucane selon l'une quelconque des revendications 1 à 3.

**6.** Composition pour utilisation en tant qu'hydratant pour la peau, comprenant en tant que substance active la composition contenant du β-glucane selon l'une quelconque des revendications 1 à 3.

**7.** Composition contenant du β-glucane selon l'une quelconque des revendications 1 à 3 pour utilisation en thérapie.

**8.** Composition contenant du β-glucane selon l'une quelconque des revendications 1 à 3 pour utilisation dans le traitement de la constipation.

**9.** Composition contenant du β-glucane selon l'une quelconque des revendications 1 à 3 pour utilisation en tant qu'immunopotentialisateur.

**10.** Utilisation d'une composition contenant du β-glucane selon l'une quelconque des revendications 1 à 3 pour la fabrication d'un médicament pour utilisation dans le traitement de la constipation ou en tant qu'immunopotentialisateur.

FIG. 1

FIG. 2

FIG. 3

FIG. 4

Test group
Comparative group 1
Comparative group 2
Control group

Days after inoculation

(Detection limit)

Bacterial counts in the spleen
(c. f. u.)

FIG. 5

FIG. 6

FIG. 7

FIG. 8

FIG. 9

FIG. 10

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP S57149301 A
- JP H54063 B
- JP 2002335926 A
- JP H6340701 A
- JP 2002204687 A
- JP 62205008 A
- JP 10310515 A
- JP 2001048796 A
- JP 2003113114 A
- JP 2003040785 A
- JP 2001323001 A
- JP 57149301 A
- JP H348201 B